(19) Europäisches Patentamt
European Patent Office
Office européen des brevets

(11) Publication number: 0 096 520 A2

(12) EUROPEAN PATENT APPLICATION

(21) Application number: 83303079.4

(22) Date of filing: 27.05.83

(51) Int. Cl.$^3$: G 01 N 33/54

(30) Priority: 03.06.82 GB 8216155
24.07.82 GB 8221458
06.01.83 GB 8300293

(43) Date of publication of application: 21.12.83 Bulletin 83/51

(84) Designated Contracting States: AT CH DE FR GB IT LI NL SE

(71) Applicant: AGROPRODUITS S.A.
CP 240
CH-1211 Geneva 25(CH)

(72) Inventor: Powell, Richard John
CP 240
1211 Geneva 25(CH)

(74) Representative: Slater, John Arthur et al,
Marks & Clerk 57-60 Lincoln's Inn Fields
LondonWC2A 3LS(GB)

# (54) Antigen detection: rheumatoid arthritis.

(57) An antigen associated with the pathogenesis of rheumatoid arthritis is detected *in vitro* in an essentially untreated human bodily fluid by a method which includes the steps of:

a) obtaining the antigen in a purified form
b) obtaining a readily detectable anti-immunoglobulin factor which shows no cross-reaction with the antigen
c) treating the antigen with the bodily fluid so that antibodies for the antigen which are present in the fluid will complex with the antigen, and
d) treating the treated antigen with the readily detectable anti-immunoglobulin factor so that the anti-immunoglobulin factor binds only to complexed antibodies, whereby the presence of bound anti-immunoglobulin factor and hence of complexed antibodies can be detected. The method can be performed using a kit which comprises a base surface having adsorbed thereupon a sample of the said antigen, and a readily detectable anti-immunoglobulin factor. In particular, the method is suited for detection of an FC antigen.

EP 0 096 520 A2

# ANTIGEN DETECTION: RHEUMATOID ARTHRITIS

The present invention relates to a method for the detection of an antigen associated with the pathogenesis of rheumatoid arthritis and hence to a method for the detection *in vitro* of rheumatoid arthritis and to a diagnostic kit for detecting a said antigen. The method of the present invention can be used to follow the course of the disease and/or its response to therapy such as for example treatment with gold, penicillamine or cytotoxic agents.

In a paper entitled "Radioimmunoassay of IgG and IgM Rheumatoid Factors reacting with Human IgG" in the Journal of Immunology, 119, July 1977, pp. 295-300, Carson *et al*. describe a method for detecting IgG and IgM rheumatoid factors. That method however is coarse and unselective and the workers found it necessary to extensively treat the serum to be analysed before subjecting it to the described method. The results obtained are far inferior to those obtainable by the method of the present invention.

According to a first aspect of the present invention there is provided a method for the *in vitro* detection in an essentially untreated human bodily fluid of an antigen associated with the pathogensis of rheumatoid arthritis which method includes the steps of:

a) obtaining the antigen in purified form,

b) obtaining a readily detectable anti-immunoglobulin

factor which shows no cross-reaction with the antigen

c) treating the antigen with the bodily fluid so that antibodies for the antigen which are present in the fluid will complex with the antigen, and

d) treating the treated antigen with the readily detectable anti-immunoglobulin factor

so that the anti-immunoglobulin factor binds only to complexed antibodies, whereby the presence of bound anti-immunoglobulin factor and hence of complexed antibodies can be detected.

By the term "essentially untreated human bodily fluid" is meant a human bodily fluid, such as serum, lymph, whole blood, or synovial fluid, which has not had a significant portion thereof removed or destroyed by a pre-treatment e.g. with unsolubilized IgG, 2-mercapto-ethanol, concanavalin A or by way of an immuno-absorption, pepsin digestion or heat treatment. The term is intended to include such a fluid which has been subjected to dilution or treated with buffer.

The term "antigen associated with the pathogenesis of rheumatoid arthritis" means essentially an Fc(RA) antigen or subfragment thereof which may have been subjected to a physical or chemical modification such as an aggregation. By the term "in purified form" is meant in a form in which it will show no cross-reaction with said readily detectable anti-immunoglobulin factor.

By the term "readily detectable anti-immunoglobulin factor" is meant an anti-immunoglobulin or fragment thereof which may be detected or rendered detectable, e.g. by radioactive labelling, the attachment of a fluorescent or chemiluminiscent marker, tagging with an enzyme, or by using magnetic particles, visible microspheres or free radicals radio-active labelling is preferred. The anti-immunoglobulin factor will of course be selected in accordance with the antibody with which it is to bind. In the case of human IgG a suitable anti-immunoglobulin factor is sheep IgG anti-human IgG Fab.

According to a second aspect of the present invention there is provided a kit for performing a method as defined hereinbefore, which comprises a base surface having adsorbed thereupon a sample of the said antigen, and a readily detectable anti-immunoglobulin factor. The kit may also comprise an agent to reduce non-specific binding (such as a buffer solution) for application to the base surface during performance of the method of the invention.

The base surface includes not only macro-surfaces such as, e.g. a tray formed with a plurality of wells or a column packed with for example Sephadex but also micro-surfaces such as, e.g. that of a latex or colloidal suspension. A preferred kit employs an irradiated multiwell P.V.C. tray manufactured by the Linbro Division of Flow Laboratories Inc. of Irvine, Scotland and Hamden, Connecticut, U.S.A.

The régime of the method of the present invention preferably resides in the steps of coating a suitably adsorbent base surface with a specified antigen whereby the antigen will adsorb on to said surface, washing the base surface with a buffer, applying the bodily fluid to

be tested to the washed base surface, whereby any antibodies to the antigen will complex with the adsorbed antigen, washing the base surface with buffer to remove any unbound antibodies, applying the anti-immunoglobulin factor, washing with buffer to remove unbound anti-immunoglobulin factor and then estimating the anti-immunoglobulin factor which has bound to the complexed antibodies and which therefore remains on said base surface, to obtain a count of the antibodies which have bound to the selected antigen and thus were present in the bodily fluid.

Preferably the buffer is a low phosphate buffer containing bovine serum albumin (BSA) or a glycine saline buffer containing BSA. Although the BSA buffer is preferred, other agents which reduce non-specific binding, such as detergents, gelatin and other forms of animal albumin, can be used.

It was indicated hereinbefore that the anti-immunoglobulin factor should show no cross-reaction with the antigen whose presence in the bodily fluid is to be detected. This may be ensured by first running the selected anti-immunoglobulin factor across the selected antigen and collecting the resultant anti-immunoglobulin factor from which those factors which will bind with the selected antigen have been removed, or by running the anti-immunoglobulin factor against highly purified immunoglobulin and collecting only that factor which binds thereto.

As indicated hereinbefore, the selected antigen should show no cross-section with the selected anti-immunoglobulin

factor. This may be ensured by running the antigen across the anti-immunoglobulin factor and collecting the resultant antigen from which those factors which will bind to the anti-immunoglobulin factor have been removed.

The present invention enables the presence of antibodies in human bodily fluid to be detected with great precision. Not only can IgG, IgM, IgA, IgD and IgE be detected and distinguished but further discrimination between sub-classes of said immunoglobulins and between the light chain type and even between particular structures of antigen binding site is now feasible.

To enable the method of the present invention to be understood, an embodiment will be explained by reference to the accompanying diagram. This diagram indicates a base surface 1 which is defined by a well in a plastics multi-well tray, as aforesaid. The surface 1 is first coated by introducing into the well a purified Fc IgG antigen which is conveniently obtained from any human blood plasma which has time-expired. The FcIgG antigen is pre-selected to have no cross-reaction with the anti-immunoglobulin factor. The antigen will be adsorbed on to the surface 1 and four such antigens are indicated in the diagram by the longer radial lines. The surface 1 is then washed with buffer, for example a low phosphate buffer with BSA. The purpose of this is to wash away antigens which have not become adsorbed and to prevent further binding to the surface 1. The buffer thus forms

a coating and is indicated in the diagram by the shorter radial lines marked BSA. The bodily fluid (serum in this case) is then placed in the well. If this contains rheumatoid factor (RF) antibodies, these antibodies will bind to the Fc IgG antigens. No other antibodies present in the serum will bind and so on washing again with buffer the surplus serum and all antibodies which have not bound will be washed away leaving only the RF antibodies marked RF in the diagram, bound to the antigens. Then a buffer containing a radioactively labelled anti-immunoglobulin factor is applied to surface 1. The radioactively labelled anti-immunoglobulin factors will only be able to bind on to the RF antibodies since they have already been preselected to have no cross-reaction with the Fc IgG antigen and so on washing again with buffer only those radioactively labelled anti-immunoglobulin factors which have bound on to the RF antibodies will remain. Then by taking count with a gamma counter the number of bound radioactive anti-immunoglobulin factors and thus the number of bound RF antibodies will be ascertained. In the diagram the anti-immunoglobulin factor is designated Sheep $(Fab')_2$IgG anti-human IgG Fab.

Embodiments of the present invention will now be described by reference to the following non-limitative Examples.

EXAMPLE 1

A: Preparation of highly purified human IgG Fc

Normal human plasma was obtained from a transfusion centre. The plasma was clotted using 40% calcium chloride (1 ml per 100 mls serum) and to prevent bacterial growth 1 ml of 10% sodium azide was added. The serum was expressed from the clot through a nylon mesh and the immunoglobulin was precipitated x3 from the serum using saturated ammonium sulphate. The final precipitate was redissolved in a minimum volume of distilled water and dialysed against PBS. The solution was next spun and the supernatant digested with papain at an enzyme; protein ratio 1 mg enzyme:100 mg protein in the presence of .01M 2 mercaproethanol and .002M $Na_2$ EDTA at 37°C for $3^{1/2}$ hours.

The digest was then separated over a G 150 column at 4°C. The Fc/Fab peak mw 50,000 was pooled. This pool was then dialysed into phosphate buffer pH 7.6 .01M and passaged through a Whatman CM 52 cellulose column using the same buffer. The Fab, but not the Fc, was retarded by this column. This Fc peak was then passaged through a Whatman DE 52 column after dialysis into .01M pHB phosphate buffer. The Fc was retained in in the column in this buffer and was eluted using 0.01M Phosphate buffer pH8 +0.3M NaCl. The eluted Fc prep. was then dialysed back into PBS and passed over an affinity column i.e. sheep IgG anti IgG Fab column, to remove any trace impurities of Fab still remaining. The purified Fc was then stored at -70°C.

Buffers used:-

CM column

need phosphate buffers pH 7.6 .01M

Stock 0.1M phosphate buffer i.e. $Na_2HPO_4$ anhydrous 3.44 Gms → 1 litre

$NaH_2PO_4 2H_2O$ .83 Gms

DE column

need phosphate buffer pH 8.0 .01M

Stock 0.1M phosphate buffer $Na_2HPO_4$ anhydrous 12.35 Gms → 1 litre

$Na_2H_2PO_4 2H_2O$ 2.03 Gms

Correct pH to 8.0 with NaoH

In a preferred embodiment the Fc is aggregated prior to use. The preferred method of achieving this is by effecting freeze drying over a perior of several hous (eg overnight) in a dilute solution in PBS phosphate buffer of the Fc. The degree of aggregation achieved may be assessed by the ability of the aggregated Fc to fix guinea pig complement in a standard anti-complementary activity assay. The aggregated Fc is considerably more effective in the method and kit of the present invention than is unaggregated Fc.

It is worth noting that the fact that freeze drying results in aggregation of the Fc is highly surprising; it would be expected that freeze drying would cause no significant changes to the Fc.

B: Preparation of $(Fab)_2$ IgG Sheep anti Hm IgG Fab

Sheep anti Hm IgG Fab purchased from Immuno Diagnostic Research Laboratories (IDRL) Birmingham, England, was treated three times with saturated ammonium sulphate to precipitate merely the immunoglobulin from the antiserum. The precipitate was resuspended in Phosphate buffered saline (PBS) pH 7.2 and then dialysed into 0.1M acetate buffer pH 4. Using an enzyme:protein ratio of 3:100, pepsin was added and the digestion allowed to proceed at 37°C for 18 hours. The digest was next dialysed into PBS and spun. The supernatant was then passed down a Sephadex G150 column and the $(Fab')_2$ peak (molecular weight 100,000) isolated and pooled.

The preparation was further purified by affinity chromatography i.e. the pooled $(Fab')_2$ peak was passed over a human IgG Fab Sepharose column. The bound material was eluted using 0.628M glycine HCl pH 2.8 and collected into 2M Tris HCl pH 8.2 on ice and then dialysed into PBS pH 7.2 0.1M.

It was then placed in aliquots at -70°C.

To ensure that the $(Fab')_2$ shows no cross-reaction with Fc (or, to put it another way, that it shows reaction only with Fab) the $(Fab')_2$ was passed, as described above, over a human IgG Fab sepharose column. The IgG Fab was obtained by the same techniques employed to obtain the Fc except that the Whatman columns were employed in the reverse order, i.e. DE52 before CM52, so as to retain Fab which was then eluted. The purity of the Fab is such that it contains substantially no Fc.

Acetate Buffer 0.1M pH 4

To make 1 litre

Glacial acetic acid 4.7 mls

Na acetate 2.45 Gms ($3H_2$ 0)

distilled water to 1 litre

TRIS Buffer 2M pH 8.2

| | | |
|---|---|---|
| In 100 mls | TRIS | 12.1 Gms |
| | 4NHCl | 11.44 mls |

Glycine HCl 0.628M pH 2.8

| | | |
|---|---|---|
| Glycine 2 molar | | 314 mls |
| HCl | 4N | 50 mls |

distilled water to 1 litre

PBS pH 7.2

| | |
|---|---|
| NaCl | 8.5 g |
| $Na_2\ HPO_4$ | 1.07 g |
| $NaH_2PO_4$ | 0.39 G |

Dist. $H_20$ to 1 litre

Labelling of sheep $(Fab')_2$ anti human $\gamma$ ($\mu$ and $\alpha$)

Reagents

Bovine serum albumin (BSA) 10% solution
Sheep $(Fab')_2$ anti-human $\gamma$ (protein)
$I^{125}$ (Na $I^{125}$) 1 mCi in 10$\mu l$ $NaI^{125}$
Chloramine T solution*
0.04 g in 25 mls of Todds buffer (see later).
Then dilute 1:10 with Todds buffer before use.
Sodium metabisulphite solution:
0.075 g of sodium metabisulphite in 25 mls Todds buffer

*Freshly made.

Buffers

1. Stock low phosphate buffer:-
10.2 g disodium hydrogen orthophosphate (anhydrous) ($Na_2HPO_4$)
4.36 g sodium dihydrogen orthophosphate ($NaH_2PO_4 2H_2O$)
87.6 g sodium chloride (NaCl).
Make up to 1 litre with distilled water.
Correct with sodium hydroxide → 0.1M Phosphate pH 7.2.

2. "Working" low phosphate buffer:-
   100 mls stock low phosphate buffer
   10 mls 10% BSA
   10 mls 10% sodium azide.

Make up to 1 litre with distilled water → 0.01M phosphate.

Coating buffer is the working buffer without any BSA added.

<u>Labelling procedure</u>:-

1. Spin protein in microfuge for 1 minute and put 20µl of supernatant into reaction tube.
2. Add 10µl of freshly made Chloramine T.
3. Add 10µl (≡ 1 MCi) $I^{125}$
4. Mix well
5. Wait 1 minute
6. Stop reaction by addition of 10µl of sodium metabisulphite solution
7. Add 50µl BSA
8. Load carefully onto a Pharmacia PD-10 column (prepacked with Sephadex G-25M and previously washed with approx. 10 mls Todds buffer) and wash through with Todds solution
9. Elute 16 x 1 ml fractions into test tubes already containing 50µl BSA
10. Pool the two hottest tubes (approx. tube Nos. 4 and 5)
11. Dilute to approx. 20 mls with working low phosphate buffer and add 100µl BSA
12. Store in lead container at $4^{o}C$.

The FcIgG antigen obtained was diluted in a low phosphate buffer to give a concentration of 15 μg/ml. A suitable low phosphate buffer is 0.01M phosphate buffer of pH 7.2.

2. A pair of adsorptive wells of a Linbro tray were coated with 50 μl this diluted antigen per well for one hour at room temperature.

3. The antigen was aspirated to dry the coating.

4. These two wells and two further identical adsorptive Linbro wells which had not been treated with antigen were then washed three times with a low phosphate buffer containing bovine serum albumin (BSA).

The plate containing the wells was then allowed to dry.

5. 50 ul of bodily fluid (in this case serum) to be tested for IgG rheumatoid factor which had been diluted to 1:160 parts by volume in the working buffer (low phosphate buffer with BSA) was introduced into both pairs of wells.

6. The plate containing these wells was incubated at approximately 4°C for approximately eighteen hours.

7. The unbound material (i.e. surplus serum and antibodies other than RF antibodies) was aspirated and the wells again washed three times with the working buffer (i.e., low phosphate buffer with BSA), with added Tween 20 (a wetting agent).

8. Each of the four wells was then treated and incubated with the previously prepared test anti-immunoglobulin factor (Sheep$(Fab')_2$ IgG anti-human IgG Fab) diluted in the aforesaid working buffer with Tween added and radioactively labelled with radioactive iodine, $I^{125}$, of substantially 60,000 c.p.m. The incubation of the wells was performed for approximately one hour. The unbound radioactive anti-immunoglobulin factor was then aspirated and the wells washed again three times with the working buffer.

9. The plate was then allowed to dry at room temperature and then cut up and the radiation from the pieces was counted by using a gamma counter.

## EXAMPLES 2 and 3

To test for the presence of IgA or IgM rheumatoid factor a precisely similar technique to that of Example 1 was employed, the only differences being that Sheep $(Fab')_2$IgG anti-human IgA or IgM respectively was used as the anti-immunoglobulin factor and that purification of the anti-immunoglobulin factor was effected using IgA or IgM respectively.

EXAMPLE 4

A latex suspension is prepared of polystyrene particles of approximately 3 microns in diameter. A known volume of this latex particle suspension, e.g. 800µλ , is vibrated sonically to remix any latex particles which may have settled.

This is then washed twice on 0.54 M glycine saline of pH 8.2. The latex particle suspension is then centrifuged and reconstituted in 20 ml of glycine saline and antigen Fc IgG (purified in the manner of Example 1) is added to a concentration of 10 mgms/ml. The suspension is mixed for approximately 30 minutes at room temperature and then the latex particles are washed twice in the glycine saline or a similar buffer. The latex particles are again reconstituted in 20 ml of 0.27M glycine saline of pH 8.2, this time containing 0.1% of bovine serum albumin (BSA) to block the surfaces of the latex particles to which the human antigen Fc IgG has not bonded. The coated latex particles in solution can now be stored provided an antimicrobial agent, for example sodium azide, has been added.

The bodily fluid under test (in this case serum) is then reacted with the coated latex particles for approximately 15 minutes. At the end of this period the latex particles are washed twice with the buffer, namely 0.27 M glycine saline of pH 8.2 containing 0.1% BSA. This washes away any of the test serum which has not bound to the

particles coated by the human antigen Fc IgC. The volume of the latex particles is then made up to the starting volume.

A non-human animal IgG antibody is prepared by inoculating a sheep with human IgG. After collection and purification of the Sheep $(Fab')_2$ IgG anti-human

IgG Fab (in the manner of Example 1), a

volume of the $(Fab')_2$ IgG sheep anti-human IgG Fab is added to the latex and after an incubation period of approximately 1 hour at room temperature the degree of latex agglutination is observed. This may be done visually or on a particle counter such as a Coulter machine which measures the sizes of the agglutinated or clumped particles. Other methods of determining the degree of agglutination may be employed such as nephelometric or turbidimetric analysis. The anti-immunoglobulin factor will only bind to coated particles to which the IgG rheumatoid factor of the test serum has bonded and so adjacent particles will agglutinate or clump together. This is because the added antibody acts as an agglutinating agent. The degree of agglutination or clumping observed is therefore proportional to the presence of the IgG rheumatoid factor.

Instead of determining the degree of agglutination or clumping, a volume of sheep antibody may be tagged before it is added to the human antigen Fc IgG, after the latex particle or surface has been reacted with the IgM

rheumatoid factor free test serum, as aforesaid. The tagging may be radiactive or the antibody may be linked with an enzyme before it is added. In the former case the degree of bonding of the antibody to the test serum which in turn has bonded to the human antigen Fc IgG coated surface is determined by a Geiger or similar counter and in the latter case the degree of said bonding is determined by first washing with the buffer and then observing a colour change after the addition of a colour reagent. The degree of radioactivity or colour change therefore gives an indication of the presence of IgG rheumatoid factor in the test serum.

Having obtained a measure of the bound antibody, a refined procedure can be used to evaluate the result. For example in Example 1 hereinbefore, the count results can be evaluated by determining an index by taking the mean of the count from the two wells to which the test serum was applied and subtracting from it the mean of the count from the two wells to which only the BSA was applied as a datum and comparing this with a similar mean difference using serum taken from, for example, four normal people whose blood does not contain an abnormal quantity of RF antibodies. This index may therefore be expressed as follows:

$$\text{Rheumatoid Factor Index} = \frac{\text{Test serum c.p.m. - BSA datum c.p.m.}}{\text{Mean (Normal c.p.m. - BSA datum c.p.m.)}}$$

The normal Rheumatoid Factor indices are defined as those results falling within three standard deviations of the mean of the normal sera.

Each multi-well plate usually has ninety-six wells although plates having more or less than ninety-six wells may be used. As two pairs of wells are required for each test, twenty-four different sera may be tested at each assay , by using a plate having ninety-six wells. Four normal sera are tested in each assay to facilitate the calculation of the RF index. A sample of blood from a patient with classical rheumatoid arthritis is run in each assay in order to validate the assay. The way in which the results are evaluated removes inter-assay variation and so results taken at different times are comparable.

The indexing procedure defined above enables meaningful results to be derived with great precision from the experimental data and in particular provides for detecting elevated levels of rheumatoid factors with great reliability.

EXAMPLE 5

The procedure of Example 1 was carried out on a considerable number of sera samples which were examined in duplicate. A wide variety of clinical diagnosis was embraced by the sera, which were supplied from hospitals, but without the clinical diagnosis being known to the persons performing the diagnostic tests until the completion thereof.

Results

The results were defined as an index as described hereinbefore rather than measuring RF in absolute amounts. The graph RF shown in Figure 2 of the accompanying drawings is provided with a horizontal line drawn at approximately 1.4 RF Index, which indicates the three standard deviation cut-off point. All normal results were defined as those falling below three standard deviations.

One hundred per cent of the results from patients with active erosive rheumatoid arthritis came above the three standard deviation cut-off point and none of the other conditions gave positive results. In other words, elevated levels of IgG Rheumatoid Factor (i.e. those values greater than 99.38% of the normal population) were confined only to the sera of patients with active erosive rheumatoid arthritis.

The ability to differentiate rheumatoid arthritis from SLE, a not uncommon clinical dilemma, was easily performed using this test. It was also

successful in distinguishing between patients suffering from rheumatoid arthritis with coincidence psoriasis and those with true psoriatic arthritis.

Results from the test demonstrated that early diagnosis of rheumatoid arthritis is possible i.e. within one month of disease onset. Clinical follow up of these early cases substantiated the diagnosis predicted by the assay.

A study on sera tested from children with active arthritic conditions has also been completed. In no conditions other than juvenile rheumatoid arthritis were IgG rheumatoid factors discovered.

A kit according to the present invention will preferably contain:

A Linbro plate Fc, either already coated onto the wells of the plate or for such coating

Buffers. 01M phos., Tween and BSA.

Radiolabelled antibody

Control positive serum

4 normal sera

Instructions.

A suitable tray is shown in Fig. 3 of the accompanying drawings in which

N1 to N4 represent normal controls

E represents elevated controls

T1 to T19 represent tests 1 to 19.

## EXAMPLE 6

Using the method of Example 1 with a kit 4 weeks old, employing a tray as shown in Fig. 3, the following results were obtained

| Tube | Counts per | Mean c.p.m. | Sample | F−B | RF Index | Interpretation |
|---|---|---|---|---|---|---|
| 1. | 2774 | | | | | |
| 2. | 2767 | 2770 | | | | |
| 3. | 707 | | Control 1. | 2103 | | |
| 4. | 628 | 667 | | | | |
| 5. | 2735 | | | | | |
| 6. | 2858 | 2796 | | | | |
| 7. | 776 | 733 | Control 2. | 2063 | | |
| 8. | 691 | | | | | |
| 9. | 2287 | 2332 | | | | |
| 10. | 2378 | | Control 3. | 1675 | | |
| 11. | 631 | 657 | | | | |
| 12. | 684 | | | | | |
| 13. | 2945 | 2826 | | | | |
| 14. | 2708 | | Control 4. | 2361 | | |
| 15. | 512 | 465 | | | | |
| 16. | 419 | | | | | |
| | | | $\overline{F_N \cdot F_B}$ | 2050 | | |
| 17. | 6396 | | | | | |
| 18. | 6639 | 6519 | Elevated Control | 5617 | 2.7 | ELEVATED |
| 19. | 940 | 900 | | | | |
| 20. | 861 | | | | | |
| 21. | 7221 | 7136 | | | | |
| 22. | 7051 | | Test 1. | 6239 | 3.0 | ELEVATED |
| 23. | 886 | 896 | | | | |
| 24. | 907 | | | | | |
| 25. | 2870 | 2809 | | | | |
| 26. | 2749 | | Test 2. | 2313 | 1.1 | NORMAL |
| 27. | 431 | 496 | | | | |
| 28. | 562 | | | | | |
| 29. | 9973 | 9829 | | | | |
| 30. | 9686 | | Test 3. | 8983 | 4.4 | ELEVATED |
| 31. | 846 | | | | | |
| 32. | 843 | | | | | |
| 33. | 145 | | Empty tube | | | Background |
| 34. | 155 | | Empty tube | | | Background |

The interpretation of the results was as follows

| RF index | Interpretation |
|---|---|
| Less than 2.0 | NORMAL |
| 2.0 and greater | ELEVATED |

N.B. Interpretation of the RF index for a test sample is decimal valid only if it has been calculated using data derived from the 4 normal controls tested on the same plate.

The reproducibility of the assay of the assay was found to be as follows

| Sample | Within assay R.F. index (mean + SD) | | Within assay CV (%) | Between assay R.F. index (mean ± SD) | | Between assay CV (%) |
|---|---|---|---|---|---|---|
| 1. | 1.05 | 0.08 | 7 | 1.12 | 0.10 | 9 |
| 2. | 2.65 | 0.10 | 4 | 2.88 | 0.33 | 11 |
| number of analyses | 20 | | | 33 | | |

CLAIMS

1. A method for the *in vitro* detection in an essentially untreated human bodily fluid of an antigen associated with the pathogenesis of rheumatoid arthritis which method includes the steps of:

a) obtaining the antigen in a purified form,

b) obtaining a readily detectable anti-immunoglobulin factor which shows no cross-reaction with the antigen

c) treating the antigen with the bodily fluid so that antibodies for the antigen which are present in the fluid will complex with the antigen, and

d) treating the treated antigen with the readily detectable anti-immunoglobulin factor

so that the anti-immunoglobulin factor binds only to complexed antibodies, whereby the presence of bound anti-immunoglobulin factor and hence of complexed antibodies can be detected.

2. A method according to Claim 1, wherein the antigen comprises an Fc fragment of a human immunoglobulin.

3. A method according to Claim 2, wherein the Fc has been aggregated.

4. A method according to Claim 3, wherein the aggregation has been effected by freeze drying.

5. A method according to Claim 1, wherein said readily detectable anti-immunoglobulin factor is selected from the group consisting of non-human animal $(Fab')_2$IgG anti-human IgG Fab, IgA, IgD, IgM and IgE.

6. A method according to Claim 5, wherein the anti-immunoglobulin factor is selected from the group consisting of sheep $(Fab')_2$IgG, anti-human IgG Fab and IgA.

7. A method according to Claim 1, wherein the anti-immunoglobulin factor is run against the selected antigen in a preliminary step to produce anti-immunoglobulin factor which shows no cross-reaction with the said antigen.

8. A method according to Claim 1, wherein the antigen is run against the anti-immunoglobulin factor in a preliminary step to produce antigen which shows no cross-reaction with the said anti-immunoglobulin factor.

9. A method according to Claim 1, wherein an agent which reduces non-specific binding is employed after each of said treatment steps.

10. A method according to Claim 1, which is effected by adsorbing the antigen on a base surface.

11. A method according to Claim 10, wherein said base surface is made of an adsorbent irradiated plastics surface.

12. A method according to Claim 1, wherein an index of performance is calculated in accordance with the following formula

$$\text{Index} = \frac{A-B}{C-B}$$

wherein

A represents a count per million of bound antibodies in the bodily fluid under test;

B represents a count per million of bound antibodies in a control not treated with the antigen; and

C represents a count per million of bound antibodies in a bodily fluid known to be free of abnormal quantities of said antigen.

13. A method for the *in vitro* detection in an essentially untreated human bodily fluid of an Fc antigen associated with the pathogenesis of rheumatoid arthritis which method includes the steps of:

a) obtaining a readily detectable anti-immunoglobulin factor which shows no cross-reaction with the antigen

b) obtaining an aggregated Fc antigen which shows no cross reaction with the anti-immunoglobulin factor

c) fixing the antigen on an adsorbent plastics surface

d) treating the antigen with the bodily fluid so that antibodies for the antigen which are present in the fluid will complex with the antigen

e) washing off unbound antibodies from the surface, and

f) treating the treated antigen with the readily detectable anti-immunoglobulin factor so that the anti-immunoglobulin factor binds only to complexed antibodies, whereby the presence of bound anti-immunoglobulin factor and hence of complexed antibodies can be detected.

14. A method according to claim 13 wherein the plastics surface is innodeated.

15. A kit for performing a method for the in vitro detection in an essentially untreated human bodily fluid of an antigen associated with the pathogenesis of rheumatoid arthritis, which kit comprises a base surface having adsorbed thereupon a sample of the said antigen, and a readily detectable anti-immunoglobulin factor.

16. A kit according to claim 15, wherein the base surface is that of an adsorbent irradiated plastics multi-well tray.

17. A kit according to claim 15, wherein the base surface is the surface of a latex suspension.

18. A kit for performing a method for the *in vitro* detection in an essentially untreated human bodily fluid of an antigen associated with the pathogenesis of rheumatoid arthritis which kit comprises

an adsorbent plastics surface

a radiolabelled anti-immunoglobulin factor which shows no cross-reaction with the antigen to be detected,

a sample of the antigen to be detected, in a form in which it will not show cross-reaction with the anti-immunoglobulin factor, for adsorption on the said plastics surface,

a control bodily fluid known to contain the antigen to be detected, and

one or more samples of a bodily fluid known not to contain the antigen to be detected.

19. A kit according to claim 18, wherein the plastics surface is irradiated polyvinyl/chlorine.

20. A kit according to claim 18, wherein the antigen is an Fc fragment of a human IgG.

21. A kit according to claim 19, wherein said Fc is aggregated.

22. A kit according to claim 18, which further includes instructions.

23. A kit according to claim 18, wherein the non-specific binding agent comprises a phosphate-containing solution of bovine serum albumen.

0096520

1/2

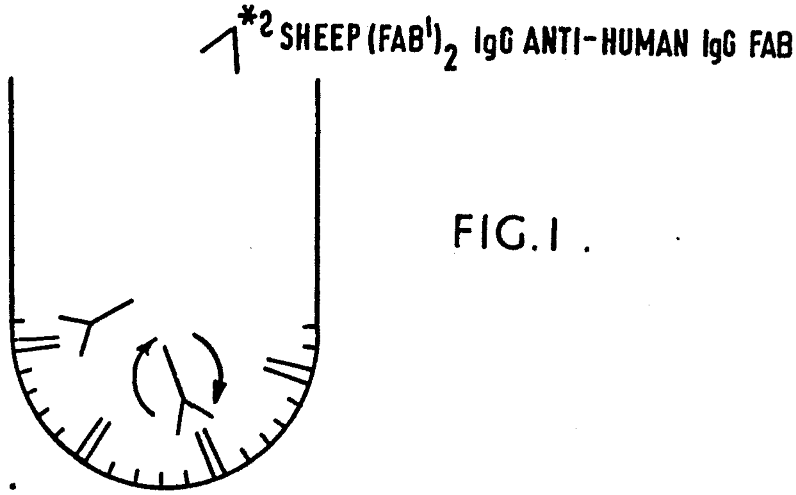


FIG. 1.

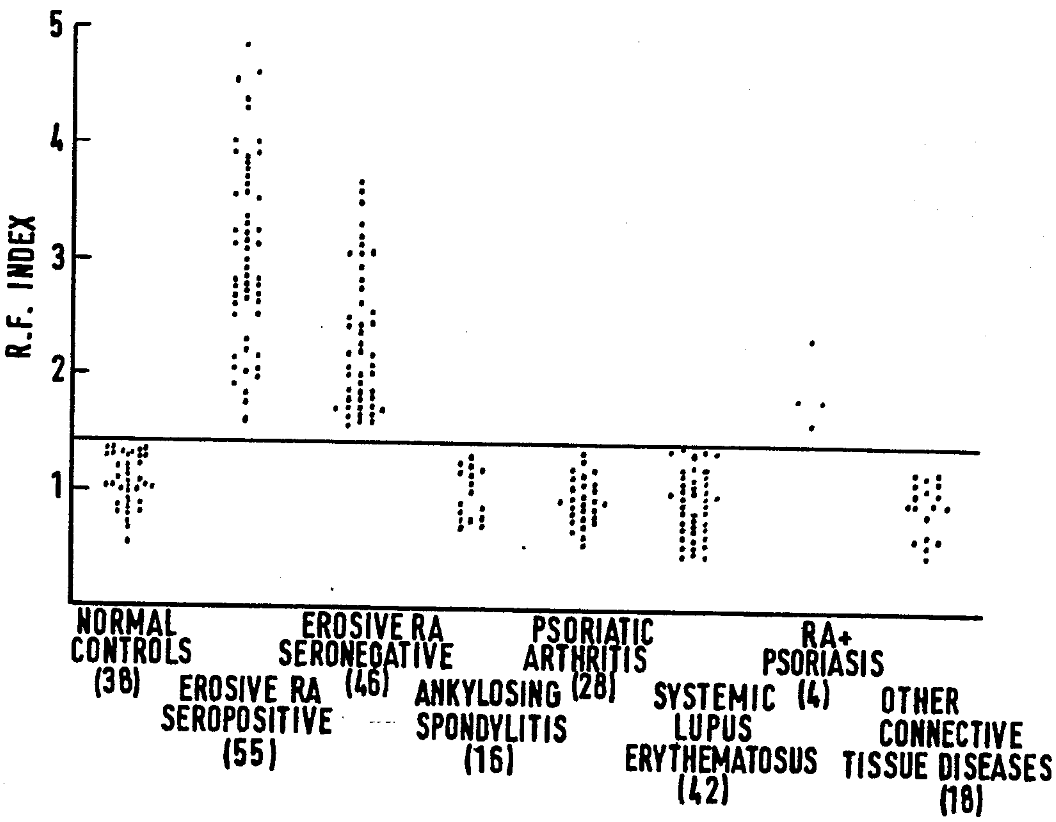


FIG. 2.

0096520

2/2

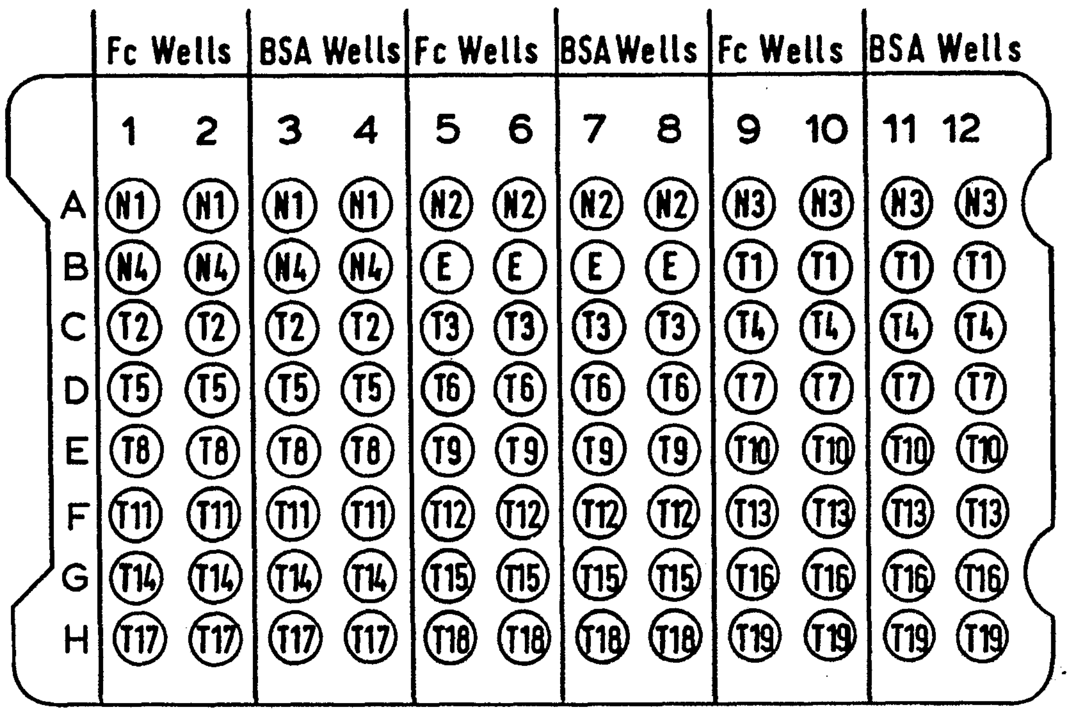


FIG. 3.